(19) Europäisches Patentamt
European Patent Office
Office européen des brevets

(11) **EP 1 357 133 B1**

(12) **EUROPÄISCHE PATENTSCHRIFT**

(45) Veröffentlichungstag und Bekanntmachung des Hinweises auf die Patenterteilung:
**17.10.2018 Patentblatt 2018/42**

(51) Int Cl.:
*C08F 8/00* (2006.01)    *A61L 15/00* (2006.01)
*B01J 20/26* (2006.01)    *A61L 15/60* (2006.01)
*C08J 3/24* (2006.01)    *C08F 8/14* (2006.01)

(21) Anmeldenummer: **03017172.2**

(22) Anmeldetag: **26.02.2000**

(54) **Verwendung pulverförmiger, vernetzter, wässrige Flüssigkeiten sowie Blut absorbierender Polymere**

Use of powdery, crosslinked polymers which absorb aqueous liquids and blood

Utilisation de polymères poudreux, réticulés, absorbant les liquides aqueux et le sang

(84) Benannte Vertragsstaaten:
**AT BE CH CY DE DK ES FI FR GB GR IE IT LI LU MC NL PT SE**

(30) Priorität: **05.03.1999 DE 19909838**

(43) Veröffentlichungstag der Anmeldung:
**29.10.2003 Patentblatt 2003/44**

(62) Dokumentnummer(n) der früheren Anmeldung(en) nach Art. 76 EPÜ:
**00907638.1 / 1 165 631**

(73) Patentinhaber: **Evonik Degussa GmbH 45128 Essen (DE)**

(72) Erfinder:
• **Mertens, Richard, Dr. 47803 Krefeld (DE)**
• **Harren, Jörg, Dr. 47807 Krefeld (DE)**

(56) Entgegenhaltungen:
EP-A- 0 233 067    EP-A- 0 521 355
EP-A- 0 695 763    EP-A- 0 856 528
EP-A- 0 882 502    EP-A- 0 889 063
WO-A-96/05234

**Beschreibung**

Superabsorber sind wasserunlösliche, vernetzte Polymere, die in der Lage sind, unter Quellung und Ausbildung von Hydrogelen große Mengen an wässrigen Flüssigkeiten und Körperflüssigkeiten, wie z. B. Urin oder Blut, aufzunehmen und unter einem bestimmten Druck zurückzuhalten. Durch diese charakteristischen Eigenschaften finden diese Polymere hauptsächlich Anwendung bei der Einarbeitung in Sanitärartikel, wie z. B. Babywindeln, Inkontinenzprodukten oder Damenbinden.

Bei den gegenwärtig kommerziell verfügbaren Superabsorbern handelt es sich im Wesentlichen um vernetzte Polyacrylsäuren oder vernetzte Stärke-Acrylsäure-Pfropfpolymerisate, bei denen die Carboxylgruppen teilweise mit Natronlauge oder Kalilauge neutralisiert sind.

Aus ästhetischen Gründen und aus Umweltaspekten besteht die zunehmende Tendenz, die Sanitärartikel wie Babywindeln, Irikontinezprodukte und Damenbinden immer kleiner und dünner zu gestalten. Um ein gleichbleibendes Gesamtretentionsvermögen der Sanitärartikel zu gewährleisten, kann dieser Anforderung nur durch Reduktion des Anteils an großvolumigen Fluff entsprochen werden. Hierdurch fallen dem Superabsorber weitere Aufgaben hinsichtlich Transport und Verteilung von Flüssigkeit zu, die sich als Permeabilitätseigenschaften zusammenfassen lassen.

Unter Permeabilität versteht man bei Superabsorbermaterialien die Fähigkeit, im gequollenen Zustand zugegebene Flüssigkeiten zu transportieren und dreidimensional zu verteilen. Dieser Prozeß läuft im gequollenen Superabsorbergel über kapillaren Transport durch Zwischenräume zwischen den Gelpartikeln ab. Ein Flüssigkeitstransport durch gequollene Superabsorberpartikel selbst folgt den Gesetzen der Diffusion und ist ein sehr langsamer Prozeß, der in der Nutzungssituation des Sanitärartikels keine Rolle bei der Verteilung der Flüssigkeit spielt. Bei Superabsorbermaterialien, die einen kapillaren Transport aufgrund mangelnder Gelstabilität nicht bewerkstelligen können, wurde durch Einbetten dieser Materialien in eine Fasermatrix eine Separation der Partikel voneinander unter Vermeidung des Gel-Blocking-Phänomens sichergestellt. In Windelkonstruktionen neuer Generation befindet sich in der Absorberschicht nur wenig oder überhaupt kein Fasermaterial zur Unterstützung des Flüssigkeitstransports. Die hier verwendeten Superabsorber müssen demnach eine ausreichend hohe Stabilität im gequollenen Zustand besitzen, damit das gequollene Gel noch eine ausreichende Menge an kapillaren Räumen besitzt, durch die Flüssigkeit transportiert werden kann.

Um Superabsorbermaterialien mit hoher Gelstärke zu erhalten, kann einerseits der Grad der Vernetzung des Polymers angehoben werden, was zwangsläufig eine Verminderung der Quellfähigkeit und des Retentionsvermögens zur Folge hat. Eine optimierte Kombination von verschiedenen Vernetzern und Comonomeren, wie in Patentschrift DE 196 46 484 beschrieben, wermag die Permeabilitätseigenschaften zwar zu verbessern, nicht aber auf ein Niveau, das beispielsweise den Einbau einer gegebenenfalls nur aus Superabsorbern bestehende Schicht in eine Windelkonstruktion erlaubt

Weiterhin können Methoden zur oberflächlichen Nachvernetzung der Polymerpartikel zur Anwendung kommen. Bei der sog. Nachvernetzung werden die Carboxylgruppen der Polymermoleküle an der Oberfläche der Superabsorberpartikel mit verschiedenen Nachvernetzungsmitteln, die mit mindestens zwei der oberflächennahen Carboxylgruppen reagieren können, zur Reaktion gebracht. Neben der Erhöhung der Gelstärke wird insbesondere die Fähigkeit zur Flüssigkeitsaufnahme unter Druck stark verbessert, da das bekannte Phänomen des Gel-Blocking unterdrückt wird, bei dem angequollene Polymerteilchen verkleben und dadurch eine weitere Flüssigkeitsaufnahme verhindert wird.

Die Oberflächenbehandlung von flüssigkeitsabsorbierenden Harzen ist bereits bekannt. Zur Verbesserung der Dispergierbarkeit wird eine ionische Komplexierung der oberflächennahen Carboxylgruppen mit polyvalenten Metallkationen in der US 4,043,952 vorgeschlagen. Die Behandlung erfolgt mit Salzen mehrwertiger Metalle, die in organischen, ggf. Wasser enthaltende Solventien, (Alkohole und andere organische Solventien), dispergiert sind.

Eine Nachbehandlung von Superabsorberpolymeren mit reaktionsfähigen, oberflächenvernetzenden Verbindungen (Alkylencarbonate) zur Erhöhung der Flüssigkeitsaufnahmefähigkeit unter Druck wird in DE-A-40 20 780 beschrieben.

EP-A-0574260 offenbart ein Beispiel 28, in Rahmen dessen ein absorbierendes Harz mit Aluminiumsulfat, Glycerin und Wasser vermischt und anschließend erhitzt wird. Messwerte hinsichtlich der Permeabilität (SFC) und Retention (TB) des so dargestellten Materials enthält EP-A-0574260 nicht.

Die EP 0 233 067 beschreibt wasserabsorbierende, an der Oberfläche vernetzte Harze, die durch Reaktion von einem superabsorbierenden Polymerpulver mit einer Aluminiumverbindung erhalten werden. Als Behandlungsiösung findet eine Mischung aus Wasser und Diolen Verwendung, die den Einsatz von niederen Alkoholen als Lösemittel überflüssig machen soll. Es werden bevorzugt 100 Teile Vernetzerlösung auf 100 bis 300 Teile Absorber aufgebracht. Gemäß der Beispiele findet die Umsetzung mit der Aluminiumkomponente bei Raumtemperatur statt. Die dem Reaktionsmedium Wasser zugefügten Diole (z. B. Polyethylenglycol 400 und 2000, 1,3-Butandiol oder 1,5-Pentandiol) dienen dazu, ein Verklumpen des Superabsorbers bei der Behandlung mit den hier verwendeten großen Mengen an wässriger Behandlungslösung zu verhindern. Das Lösemittel wird in einer anschließenden Trocknung bei 100°C entfernt. Die so behandelten Polymere weisen ein nicht ausreichendes Eigenschaftsniveau auf, wobei eine Verbesserung der Absorptionsfähigkeit unter Druck nicht erreicht wird. Außerdem ist eine Behandlung mit großen Mengen Behandlungslösung bei modernen, kontinuierlich arbeitenden Verfahren nicht ökonomisch durchführbar.

In der WO 96/05234 wird ein Verfahren zur Herstellung superabsorbierender Polymere beschrieben, gemäß dem an der Oberfläche der mindestens 10 Gew.% Wasser enthaltenden Absorberteilchen eine vernetzte Schicht durch eine Reaktion von einem reaktiven, hydrophilen Polymeren oder einer reaktiven metallorganischen Verbindung mit einem mindestens bifunktionellen Vernetzer unter 100°C gebildet wurde. Die Polymerisate sollen ein ausgewogenes Verhältnis von Absorption, Gelfestigkeit und Permeabilität aufweisen, wobei die Meßwerte nach äußerst niedrigen. Bewertungskriterien ermittelt werden. So werden beispielsweise die Absorption und die Permeabilität ohne jegliche Druckbelastung bestimmt. Nachteilig ist bei diesem bekannten Verfahren die Verwendung von Lösemitteln und toxisch bedenklichen Vernetzungsreagentien wie z.B, den als bevorzugt genannten Polyiminen, alkoxylierten Silikon- bzw. Titan-Verbindungen und Epoxiden.

Durch eine entsprechende Behandlung von kommerziell erhältlichen Superabsorberprodukten mit Aminopolymeren in organischen Lösungsmitteln wird in WO 95/22356 und WO 97/12575 eine Verbesserung der Permeabilitäts- und Flüssigkeitstransporteigenschaften erreicht. Der gravierende Nachteil des hier beschriebenen Verfahrens liegt neben der Verwendung von toxikologisch bedenklichen Polyaminen und Polyiminen in dem Einsatz großer Mengen organischer Lösungsmittel, die für die Behandlung der Polymere notwendig sind. Der damit verbundene Sicherheitsaspekt und Kostenaufwand schließt eine großtechnische Produktion aus. Neben der toxikologischen Bedenklichkeit dieser Behandlungsmittel ist weiterhin zu berücksichtigen, daß sie unter den hohen Nachvernetzungstemperaturen auch zur Zersetzung neigen, was sich u.a. in einer Gelbfärbung der Absorberpartikel äußert.

Einen Hinweis darauf, daß unter Beibehaltung einer hohen Retentionskapazität und Aufnahmefähigkeit von Flüssigkeit unter Druck bei der Nachvernetzungsstufe ebenfalls die Permeabilitätseigenschaften drastisch gesteigert werden können, ist aus dem vorstehend beschriebenen Stand der Technik nicht zu erkennen.

Aufgabe der vorliegenden Erfindung war es daher, superabsorbierende Polymere bereitzustellen, die als Eigenschaftkombination nicht nur eine hohe Aufnahmekapazität unter Druck, sondern auch die üblicherweise gegenläufigen Eigenschaften eines hohen Retentionsvermögens und einer guten Permeabilität in sich vereinigen, d. h. ein Niveau der Eigenschaftskombination aufweisen, bei dem neben einem Retentionswert von $\geq$ 25 g/g mindestens ein SFC-Wert von mindestens 30 $10^{-7}$, vorzugsweise von mindestens 50 · $10^{-7}$ cm$^3$s/g vorliegt. Insbesondere lag die Aufgabe darin, superabsorbierende Polymere zur Verfügung zu stellen, die sich vor allem für die Verwendung in sehr dünnen Windelkonstruktionen mit sehr hohem Superabsorberanteil eignen. Für diesen Fall sind insbesondere Polymere mit Retentionswerten von $\geq$ 25 g/g und Permeabilitätswerte von SFC > 70 · $10^{-7}$ cm$^3$ s /g erforderlich.

Eine weitere Aufgabe der Erfindung war es, Herstellungsverfahren für solche superabsorbierenden Polymeren zu finden, die einfach, ökonomisch und sicher durchführbar sind, eine gleichmäßige Produktqualität liefern und bei denen insbesondere niedrige Lösungsmittelmengen verwendet und organische Lösungsmittel nach Möglichkeit vermieden werden. Darüber hinaus sollen die Verfahren ohne die Verwendung toxikologisch bedenklicher Substanzen durchführbar sein.

Die erfindungsgemäße Aufgabe wird durch den Gegenstand des Anspruch 1 gelöst.

Überraschenderweise ergibt sich nämlich durch die Beschichtung eines teilchenförmigen Absorberharzes mit einer wässrigen Lösung eines Polyols, das mit den oberflächennahen Molekülgruppen, vorzugsweise mit den Carboxylgruppen, in Anwesenheit eines Kations einer Salzkomponente unter Erhitzung auf 150 bis 300 °C reagiert hat, ein Superabsorberharz mit einer signifikanten Verbesserung der Permeabilitätseigenschaften bei sehr gutem Retentionsvermögen.

Völlig unerwartet führt die wässrige Lösung der Kombination von Nachvernetzer-Komponenten zum erwünschten Ergebnis, nämlich Superabsorberharzen mit einem hohen Retentionsvermögen auch unter Druck bei gleichzeitig ausgezeichneten Permeabilitätseigenschaften. Eine aufeinander folgende separate Anwendung sowohl einer wässrigen Lösung des organischen Nachvernetzungsmittels bzw. der wässrigen Salzlösung mit jeweiligem Erhitzen führt nicht zu einer vergleichbar guten Produktcharakteristik.

Die alleinige Verwendung eines Polyols als organisches Nachvernetzungsmittel in wässriger Lösung führt zu Produkten mit hoher Retentionskapazität, hoher Gelstärke und hohem Aufnahmevermögen unter Druck. Eine signifikante Steigerung der Permeabilität im gequollenen Zustand kann allerdings nur durch einen entsprechend höheren Grad der Vernetzung der Polymere bei der Polymerisation, bzw. einer stärkeren Nachvernetzung (erhöhte Mengen Nachvernetzungsmittel oder drastischere Bedingungen) und dem damit verbundenen Verlust an Retentionskapazität erreicht werden.

Die alleinige Nachvernetzung mit Kationen hoher positiver Ladungsdichte führt ebenfalls nicht zu Polymerisaten mit der erwünschten Eigenschaftskombination. Insbesondere lassen sich keine befriedigenden Werte bei der Flüssigkeitsaufnahme unter Druck und keine guten Permeabilitätseigenschaften erreichen. Die Behandlung von Superabsorberpolymeren nur mit mehrwertigen Kationen vermag daher nur die Geschwindigkeit der Flüssigkeitsaufnahme zu erhöhen. Eine Verbesserung der Druckstabilität oder gar der Flüssigkeitstransporteigenschaften im gequollenen Zustand wird nicht erreicht.

Erfindungsgemäß werden als organische Nachvernetzer-Komponente e) Polyole eingesetzt, die mit den Oberflächen COOH-Gruppen des Polymerisats reagieren.

Vorzugsweise werden als Polyole aliphatische Polyhydroxyverbindungen, wie $C_2$-$C_8$-Alkylendiole, wie z, B. Ethylenglykol, 1,3-Propandiol, 1,4-Butandiol, 1,5-Pentandiol, 1,6-Hexandiol, Dianhydrosorbit, $C_2$-$C_8$-Alkylentriole, wie z. B. Glycerin, Trimethylolpropan, höherfunktionelle Hydroxyverbindungen, wie z. B. Pentaerythrit und Zuckeralkohole, wie z. B. Sorbit sowie Di- und Polyalkylenglykole, wie z. B. Diethylenglykol, Dipropylenglykol, Triethylenglykol,. Tetraethylenglykol, Tetrapropylenglykol, Polyethylenglykol, Polypropylenglykol, Polyglykole auf Basis von 2 oder mehr unterschiedlichen Monomereinheiten, wie z. B. ein Polyglykol aus Ethylenoxid- und Propylenoxideinheiten, verwendet. Die organische Nachvernetzerkomponente bzw. deren Mischungen werden in Mengen von 0,01 - 5 Gew.%, bevorzugt 0,1 -2,5 Gew.% und besonders bevorzugt von 0,5 bis 1,5 Gew.%, bezogen auf das zu vernetzende Polymerisat, eingesetzt.

Erfindungsgemäß werden als Komponente f) vorzugsweise wässrige Lösungen von Salzen zur Vernetzung der oberflächennahen Carboxylatgruppen eingesetzt, deren Anionen Chloride, Bromide, Sulfate, Carbonate, Nitrate, Phosphate oder organische Anionen wie Acetate und Lactate sind. Die Kationen leiten sich vorzugsweise von ein- und mehrwertigen Kationen ab, die einwertigen insbesondere von Alkalimetallen, wie Kalium, Natrium, Lithium, wobei Lithium bevorzugt wird. Erfindungsgemäß verwendete zweiwertige Kationen leiten sich von Zink, Beryllium, Erdalkalimetallen wie Magnesium, Calzium, Strontium ab, wobei Magnesium bevorzugt wird. Weitere Beispiele für erfindungsgemäß einsetzbare höherwertige Kationen sind Kationen von Aluminium, Eisen, Chrom, Mangan, Titan, Zirkonium und andere Übergangsmetalle sowie Doppelsalze solcher Kationen oder Mischungen der genannten Salze. Bevorzugt werden Aluminiumsalze und Alaune und deren unterschiedliche Hydrate wie z.B. $AlCl_3$ x 6 $H_2O$, $NaAl(SO_4)_2$ x 12 $H_2O$, $KAl(SO)_4$ x 12 $H_2O$ oder $Al_2(SO_4)_3$ x 14 -18 $H_2O$ eingesetzt. Besonders bevorzugt werden $Al_2(SO_4)3$ und seine Hydrate verwendet. Eingesetzt wird die Salzkomponente, berechnet auf das Kation, in Mengen von 0,001 -1,0 Gew.%, bevorzugt 0,005 - 0,5 Gew.%, und besonders bevorzugt 0,01 -0,2 Gew.%, bezogen auf das Polymerisat.

Das wasserabsorbierende Polymerisat, das oberflächenvernetzt wird, wird durch Polymerisation von a) 55-99,9 Gew% eines einfach ungesättigten Monomeren mit Säuregruppen erhalten. Hierbei sind carboxylgruppenhaltige Monomere bevorzugt, wie z.B. Acrylsäure, Methacrylsäure oder 2-Acrylamido-2-methylpropansulfonsäure oder Mischungen dieser Monomeren.. Es ist bevorzugt, daß mindestens 50% und besonders bevorzugt mindestens 75% der Säuregruppen Carboxyl-Gruppen sind. Die Säuregruppen sind zu mindestens zu 25 Mol% neutralisiert, d.h. liegen als Natrium-, Kalium- oder Ammoniumsalze vor. Bevorzugt liegt der Neutralisationsgrad bei mindestens 50 mol%. Besonders bevorzugt ist ein Polymerisat, das durch Polymerisation von Acrylsäure oder Methacrylsäure, deren Carboxylgruppen zu 50-80 Mol% neutralisiert ist, in Gegenwart von Vernetzern erhalten wurde.

Als weitere Monomere b) können für die Herstellung der absorbierenden Polymerisate 0-40 Gew% ethylenisch ungesättigte mit a) copolymerisierbarer Monomere, wie z. B. Acrylamid, Methacrylamid, Hydroxyethylacrylat, Dimethylaminoalkyl(meth)-acrylat, Dimethylaminopropylacrylamid oder Acrylamidopropyltrimethylammoniumchlorid verwendet werden. Über 40 Gew% dieser Monomerem können die Quellfähigkeit der Polymerisate verschlechtern.

Als Vernetzerkomponente c), die während der Polymerisation von a) und b) vorhanden ist, können alle Verbindungen verwendet werden, die mindestens zwei ethylenisch ungesättigte Doppelbindungen oder eine ethylenisch ungesättigte Doppelbindung und eine gegenüber Säuregruppen der Monomeren a) reaktive funktionelle Gruppe oder mehrere gegenüber Säuregruppen reaktive funktionelle Gruppen tragen. Beispielhaft seien genannt: aliphatische Amide wie z. B. das Methylenbisacryl-bzw. -methacrylamid oder Ethylenbisacrylamid, ferner aliphatische Ester von Polyolen oder alkoxylierten Polyolen mit ethylenisch ungesättigten Säuren, wie Di(meth)acrylate oder Tri(meth)acrylate, Butandiol- oder Ethylenglykol, Polyglykolen, Trimethylolpropan, Di- und Triacrylatester des, vorzugsweise mit 1 bis 30 Mol Alkylenoxid oxalkylierten, vorzugsweise ethoxylierten Trimethylolpropans, Acrylat- und Methacrylatester von Glycerin und Pentaerythrit, sowie des mit vorzugsweise 1 bis 30 Mol Ethylenoxid oxethylierten Glycerins und Pentaerythrits, ferner Allylverbindungen wie Allyl(meth)acrylat, alkoxyliertes Allyl(meth)acrylat mit vorzugsweise 1 bis 30 Mol Ethylenoxid umgesetzt, Triallylcyanurat, Triallylisocyanurat, Maleinsäurediallylester, Polyallylester, Tetraallyloxiethan, Triallylamin, Tetraallylethylendiamin, Allylester der Phosphorsäure bzw. phosphorigen Säure, ferner vernetzungsfähige Monomere, wie N-Methylolverbindungen von ungesättigten Amiden wie von Methacrylamid oder Acrylamid und die davon abgeleiteten Ether. Mischungen der genannten Vernetzer können ebenfalls eingesetzt werden. Der Anteil an den vernetzenden Comonomeren liegt bei 0,1 bis 5 Gew%, bevorzugt bei 0,01 bis 3,0 Gew%, bezogen auf die Gesamtmenge der Monomeren.

Als wasserlösliche Polymere d) können in den absorbierenden Polymerisaten 0-30 Gew.% wasserlösliche Polymerisate, wie teil- oder vollverseifter Polyvinylalkohol, Polyvinylpyrrolidon, Stärke oder Stärkederivate, Polyglycole oder Polyacrylsäuren enthalten, vorzugsweise einpolymerisiert sein. Das Molekulargewicht dieser Polymeren ist unkritisch, solange sie wasserlöslich sind. Bevorzugte wasserlösliche Polymere sind Stärke und Polyvinylalkohol. Der bevorzugte Gehalt an solchen wasserlöslichen Polymeren im erfindungsgemäß absorbierenden Polymerisat liegt bei 0-30 Gew.%, vorzugsweise 0-5 Gew%, bezogen auf die Gesamtmenge der Komponenten a) bis d). Die wasserlöslichen Polymere, vorzugsweise synthetische, wie Polyvinylalkohol, können auch als Pfropfgrundlage für die zu polymerisierenden Monomeren dienen.

Zur Initiierung der radikalischen Polymerisation werden die gebräuchlichen Initiatoren wie z.B. Azo- oder Peroxoverbindungen, Redoxsysteme oder UV-Initiatoren (Sensibilisatoren) verwendet.

Die Herstellung der Polymerisate erfolgt vorzugsweise nach zwei Methoden:

Nach der ersten Methode wird das teilneutralisierte Monomere a), vorzugsweise die Acrylsäure in wäßriger Lösung in Gegenwart von Vernetzern und ggf. weiteren Komponenten durch radikalische Polymerisation in ein Gel überführt, das zerkleinert, getrocknet, gemahlen und auf die gewünschte Partlkelgröße abgesiebt wird. Diese Lösungspolymerisation kann kontinuierlich oder diskontinuierlich durchgeführt werden. Der Stand der Technik weist ein breites Spektrum an Variationsmöglichkeiten hinsichtlich der Konzentrationsverhältnisse, Temperaturen, Art und Menge der Initiatoren aus, Typische Verfahren sind in den folgenden Veröffentlichungen beschrieben: US 4 286 082, DE 27 06 135 und US 4 076 663

Auch die inverse Suspensions- und Emulsionspolymerisation kann zur Herstellung der Produkte angewendet werden. Gemäß diesen Prozessen wird eine wäßrige, teilneutralisierte Lösung der Monomeren a), vorzugsweise Acrylsäure mit Hilfe von Schutzkolloiden und/oder Emulgatoren in einem hydrophoben, organischen Lösungsmittel dispergiert und durch Radikalinitiatoren die Polymerisation gestartet. Die Vernetzer sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren d) als Pfropfgrundlage über die Monomerlösung oder durch direkte Vorlage in die Ölphase. Anschließend wird das Wasser azeotrop aus dem Gemisch entfernt und das Polymerisat abfiltriert und ggf. getrocknet. Die Vernetzung kann durch Einpolymerisation eines in der Monomerenlösung gelösten polyfunktionellen Vernetzers und/oder durch Reaktion geeigneter Vernetzungsmittel mit funktionellen Gruppen des Polymeren während der Polymerisationsschritte erfolgen. Die Verfahren sind beispielsweise in den Veröffentlichungen US 43 40 706, DE 3713 601, DE 28 40 010 und WO 96/05234 beschrieben.

Die Trocknung des Polymerisatgels erfolgt bis zu einem Wassergehalt von 0,5-25 Gew.%, vorzugsweise von 1 bis 10 Gew.%, besonders bevorzugt 1 bis 8 Gew.% bei Temperaturen, die üblicherweise im Bereich von 100 - 200 °C liegen.

Hinsichtlich der Teilchenform gibt es keine besonderen Einschränkungen. Das Polymerisat kann in Form von Kügelchen vorliegen, die durch inverse Suspensionspolymerisation erhalten wurden, oder in Form von unregelmäßig geformten Teilchen, die durch Trocknung und Pulverisierung der Gelmasse aus der Lösungspolymerisation stammen. Die Teilchengröße liegt normalerweise unter 3000 $\mu$m, bevorzugt zwischen 20 und 2000 $\mu$m, und besonders bevorzugt zwischen 150 und 850 $\mu$m.

Die werden in Form ihrer wässrigen Lösungen aufgebracht. Geeignete Lösungsmittel sind Wasser und ggf. polare, mit Wasser mischbare organische Lösungsmittel wie beispielsweise Aceton, Methanol, Ethanol oder 2-Propanol bzw deren Gemische. Der Begriff wäßrige Lösung im Sinne der Erfindung bedeutet in Bezug auf die Lösungsmittelkomponente, daß neben dem Wasser auch noch andere organische Lösungsmittel enthalten sein können. Die Konzentration der jeweiligen Nachvernetzerkomponente in dem wässrigen Lösungsmittel kann in weiten Grenzen schwanken und liegt im Bereich von 1 bis 80 Gew,%, vorzugsweise im Bereich von 5 bis 65 Gew.% und ganz besonders bevorzugt in einem Bereich von 10 bis 40 Gew.%. Das bevorzugte Lösungsmittel für die Polyole als Nachvernetzungsmittel bzw. die Salzkomponente ist Wasser, das in einer Menge von 0,5 -10 Gew.%, bevorzugt 0,75 - 5 Gew.%, und besonders bevorzugt 1,0 - 4 Gew.%, bezogen auf das Polymerisat verwendet wird.

Sofern das Polyol und die Salzkomponente in einer wässrigen Lösung vorliegen, können die lösbaren Mengen beider Komponeten durch Aussalzeffekte begrenzt sein und sind entsprechend der Zusammensetzung anzupassen. Da aus sicherheitstechnischen Gründen zur Vermeidung von Explosionen die Menge an organischem Solvens so gering wie möglich gehalten werden soll, ist eine stabile Mischphase Wasser/organisches Lösungsmittel/Polyole/Salzkomponete nicht über beliebige Konzentrationen der Verbindung zu erreichen. Eine bevorzugte Lösung besteht beispielsweise aus 1,5 - 3 Gew. Teilen Wasser, 0,5 - 1 Gew.Teilen Polyolkomponente und 0,4 - 0,6 Gew. Teilen eines anorganischen Salzes. Die gesamte Menge an Lösungsmittel wird üblicherweise im Bereich von 0,5 - 12 Gew.%, bevorzugt bei 1 - 7 Gew.% und besonders bevorzugt bei 1 - 5 Gew.% bezogen auf das Polymerisat eingesetzt.

Um die Flüssigkeitsmengen, die auf das Polymerisat aufgebracht werden, zu reduzieren, können neben Wasser und den oben genannten organischen Solventien auch andere Lösungsvermittler zum Einsatz kommen, wie zum Beispiel anorganische oder organische Säuren oder Komplexbildner.

Abhängig von der Löslichkeit der beiden Komponenten e) und f) wird die Lösung vor dem Aufbringen auf das Polymerisat auf 20-100 °C, bevorzugt auf 20-60 °C erwärmt. Ein getrenntes, aber gleichzeitiges Zudosieren von einer Lösung des Polyols und einer Lösung der Salzkomponente ist ebenfalls möglich, wenn eine homogene Verteilung beider Komponenten auf dem Polymerisat gewährleistet ist und das Material anschließend thermisch nachbehandelt wird. Bevorzugt ist das Aufbringen einer einzigen Lösung auf das Polymerisat, in der beide Komponenten gelöst sind.

Die Nachvernetzerlösung sollte sehr gut mit den Polymerteilchen vermischt werden. Geeignete Mischaggregate zum Aufbringen der Nachvernetzerlösung sind z.B. Patterson-Kelley-Mischer, DRAIS-Turbulenzmischer, Lödigemischer, Ruberg-Mischer, Schneckenmischer, Tellermischer und Wirbelschichtmischer sowie kontinulerlich arbeitende senkrechte Mischer, in denen das Polymerisat-Pulver mittels rotierender Messer in schneller Frequenz gemischt wird (Schugi-Mischer). Es besteht auch die Möglichkeit, die Beschichtung des Polymerisates während eines Verfahrensschrittes bei der Herstellung des Polymerisates vorzunehmen. Hierzu ist besonders der Prozeß der inversen Suspensionspolymerisation geeignet.

Nachdem die Nachvernetzerlösung mit den Polymerteilchen vermischt worden ist, erfolgt die Nachvernetzungsreaktion bei Temperaturen im Bereich von 150°C bis 300°C, bevorzugt >150°C bis 250°C und besonders bevorzugt 160°C bis 210°C. Die optimale Zeitdauer der Nacherhitzung kann für die einzelnen Vernetzertypen mit wenigen Versuchen leicht ermittelt werden. Sie wird dadurch begrenzt, wenn das gewünschte Eigenschaftsprofil des Superabsorbers infolge von Hitzeschädigung wieder zerstört wird. Die thermische Behandlung kann in üblichen Trocknern oder Öfen durchgeführt werden; beispielhaft seien Drebrohröfen, Wirbelbetttrockner, Tellertrockner, Paddeltrockner oder Infrarottrockner genannt.

Die Polymeren können in großtechnischer Weise nach bekannten Verfahren kontinuierlich oder diskontinuierlich hergestellt werden.

Die Polymerisate können für weite Anwendungsgebiete eingesetzt werden. Wenn sie z. B. als Absorbierungsmittel in Damenbinden, Windein oder in Wundabdeckungen verwendet werden, besitzen sie die Eigenschaft, daß sie große Mengen an Menstrationsblut, Urin oder anderen Körperflüssigkeiten schnell absorbieren. Da die Mittel die absorbierten Flüssigkeiten auch unter Druck zurückhalten und zusätzlich in der Lage sind, im gequollenen Zustand weitere Flüssigkeit innerhalb der Konstruktion zu verteilen, werden sie besonders bevorzugt in höheren Konzentrationen, in Bezug auf das hydrophile Fasermaterial wie z.B. Fluff eingesetzt als dies bisher möglich war. Sie eignen sich auch für den Einsatz als homogene Superabsorberschicht ohne Fluffanteil innerhalb der Windelkonstruktion, wodurch besonders dünne Windein möglich sind. Weiterhin eignen sich die Polymere zum Einsatz in Hygieneartikel (Inkontinenzprodukte) für Erwachsene.

Die Polymeren werden auch in Absorberartikeln eingesetzt, die für die verschiedensten Verwendungen geeignet sind, so z.B. durch Mischen mit Papier oder Fluff oder synthetischen Fasern oder durch Verteilen der Superabsorber zwischen Substraten aus Papier, Fluff oder nicht gewebten Textilien oder durch Verarbeitung in Trägermaterialien zu einer Bahn. Desweiteren finden die Polymeren auch überall dort Verwendung, wo wässrige Flüssigkeiten absorbiert werden müssen, wie z. B. bei Kabelummantelungen, in Lebensmittelverpackungen, im Agrarbereich bei der Pflanzenaufzucht und als Wasserspeicher sowie als Wirkstoffträger mit einer zeitlich verzögerten Freisetzung des Wirkstoffes an die Umgebung.

Die Superabsorber zeigen überraschenderweise eine bedeutende Verbesserung der Permeabilität, d. h. eine Verbesserung des Flüssigkeitstransportes im gequollenen Zustand. Es werden Polymerisate mit Permeabilitäts-Werten (SFC) von bis zu $70 \cdot 10^{-7}$ cm$^3$ s/g bei einer Retention (TB) von mindestens 27 g/g erhalten, vorzugsweise Polymere mit SFC-Werten von $> 70 \cdot 10^{-7}$ bis $\geq 150 \cdot 10^{-7}$ cm$^3$ s/g bei einer Retention (TB) von mindestens 25 g/g. Neben diesen ausgezeichneten SFC- und Retentionswerten zeigen die erfindungsgemäßen Polymere Meßwerte für die Flüssigkeitsaufnahme unter Druck (AAP 0,7) von mindestens 18 g/g.

Die Produkte mit dieser hervorragenden Eigenschaftskombination aus sehr hohen SFC-Werten, hoher Retention und hoher Absorption unter Druck können ohne die Verwendung toxikologisch bedenklicher Substanzen hergestellt werden.

Wie aus den folgenden Beispielen zu entnehmen ist, ist die Nachvernetzung auf chemisch verschieden aufgebaute, absorbierende Polymerisate anwendbar. Damit entfällt die Notwendigkeit, bereits während der Herstellung der ·Polymerisate auf spezielle Vernetzerkombinationen, Comonomere oder aufwendige Nachbehandlungsverfahren zurückgreifen zu müssen, um eine auch nur etwas erhöhte Permeabilität zu erreichen.

**Testmethoden**

Zur Charakterisierung der erfindungsgemäßen, absorbierenden Polymerisate werden Retention (TB), Aufnahme unter Druck (AAP) und die Durchlässigkeit für 0,9%ige Kochsalzlösung im gequollenen Zustand (SFC) bestimmt.

a) Die Retention wird nach der Teebeutelmethode und als Mittelwert aus drei Messungen angegeben. Etwa 200 mg Polymerisat werden in einen Teebeutel eingeschweißt und für 30 Minuten in 0,9%ige NaCl-Lösung getaucht. Anschließend wird der Teebeutel in einer Schleuder (23 cm Durchmesser, 1.400 Upm) 3 Minuten geschleudert und gewogen. Einen Teebeutel ohne wasserabsorbierendes Polymerisat läßt man als Blindwert mitlaufen.

$$\text{Retention} = \text{Auswaage-Blindwert/Einwaage [g/g]}$$

b) Flüssigkeitsaufnahme unter Druck (AAP-Test, gemäß EP 0 339 461)
Die Aufnahme unter Druck (Druckbelastung 50 g/cm$^2$) wird nach einer in der EP 0339461, Seite 7, beschriebenen Methode bestimmt. In einen Zylinder mit Siebboden werden ca. 0,9 g Superabsorber eingewogen. Die gleichmäßig aufgestreute Superabsorberlage wird mit einem Stempel belastet, der einen Druck von 50 g/cm$^2$ ausübt. Der zuvor

gewogene Zylinder wird anschließend auf eine Glasfilterplatte gestellt, die sich in einer Schale mit 0,9%iger NaCl-Lösung befindet, deren Flüssigkeitniveau genau der Höhe der Filterplatte entspricht. Nachdem man die Zylindereinheit 1 Stunde lang 0,9%ige NaCl-Lösung saugen gelassen hat, wird diese zurückgewogen und der AAP wie folgt berechnet:

$$AAP = Auswaage\ (Zylindereinheit + Superabsorber) - Einwaage\ (Zylindereinheit + vollgesogener\ Superabsorber)\ /\ Einwaage\ Superabsorber$$

c) Permeabilität im gequollenen Zustand (SFC-Test, gemäß WO 95/22356)

In einen Zylinder mit Siebboden werden ca. 0,9 g Superabsorbermaterial eingewogen und sorgfältig auf der Siebfläche verteilt. Das Superabsorbermaterial läßt man in JAYCO synthetischen Urin [Zusammensetzung: 2,0 g Kaliumchlorid; 2,0 g Natriumsulfat; 0,85 g Ammoniumdihydrogenphosphat; 0,15 g Ammoniumhydrogenphosphat; 0,19 g Calciumchlorid; 0,23 g Magnesiumchlorid als wasserfreie Salze in 1 l destilliertem Wasser gelöst] 1 Stunde lang gegen einen Druck von 20 $g/cm^2$ quellen. Nach Erfassung der Quellhöhe des Superabsorbers läßt man bei konstantem hydrostatischem Druck 0,118 M NaCl-Lösung aus einem nivellierten Vorratsgefäß durch die gequollene Gelchicht laufen. Die gequollene Gelschicht ist während der Messung mit einem speziellen Siebzylinder abgedeckt, der eine gleichmäßige Verteilung der 0,118 M NaCl-Lösung oberhalb des Gels und konstante Bedingungen (Meßtemperatur 20-25°C) während der Messung bezüglich der Gelbettbeschaffenheit gewährleistet. Der auf den gequollenen Superabsorber wirkende Druck ist weiterhin 20 $g/cm^2$. Mit Hilfe eines Computers und einer Waage wird die Flüssigkeitsmenge, die die Gelschicht als Funktion der Zeit passiert in Intervallen von 20 Sekunden innerhalb einer Zeitperiode von 10 Minuten erfaßt. Die Fließrate g/s durch die gequollene Gelschicht wird mittels Regressionsanalyse mit Extrapolation der Steigung und Ermittlung des Mittelpunkts auf den Zeitpunkt t=0 der Fließmenge innerhalb der Minuten 2-10 ermittelt. Die Berechnung des SFC-Wertes (K) berechnet sich wie folgt:

$$K = \frac{F_s(t=0) \cdot L_0}{r \cdot A \cdot \Delta P} = \frac{F_s(t=0) \cdot L_0}{139506}$$

Wobei:

$F_s$ (t = 0)   die Fließrate in g/s
$L_0$   die Dicke der Gelschicht in cm
r   die Dichte der NaCl-Lösung (1,003 $g/cm^3$)
A   die Fläche der Oberseite der Gelschicht im Meßzylinder (28,27 $cm^2$)
$\Delta P$   der hydrostatische Druck, der auf der Gelschicht lastet (4920 $dyne/cm^2$)

und

K   der SFC-Wert ist [$cm^3 \cdot s \cdot g^{-1}$]

Die formale Addition der Zahlenwerte der Teebeutelretention und des SFC-Wertes verdeutlicht den sprung-haften Anstieg dieser Eigenschaftskombination bei den erfindungsgemäßen Polymerisaten im Vergleich zu unbehandeltem Superabsorberpulver oder Produkten die nach bekannten Methoden oberflächlich nachvernetzt wurden. Der Zahlenwert wird bei den erfindungsgemäßen Produkten nicht durch einen hohen Beitrag einer der beiden Werte erreicht (z. B.eines hohen TB-Retentionswertes und eines niedrigen SFC-Wertes und umgekehrt).

**Beisplele**

In den Beispielen und Vergleichsbeispielen wurde das für die Oberflächenvernetzung vorgesehene Pulver jeweils auf eine Teilchengröße von 150 $\mu$m bis 850 $\mu$m abgesiebt.

**Beispiel 1**

Eine vernetzte Polyacrylsäure (Pulver A) wurde durch einen Herstellungsprozeß gewonnen, bei dem der Gehalt an Acrylsäure, die zu 70% neutralisiert war, in der wäßrigen Monomerenlösung 26 Gew.% betrug und mit 0,7 Gew.%, bezogen auf Acrylsäure, einer Mischung aus zwei Vernetzern Triallylamin und Polyethylenglykoldiacrylat vernetzt wurde.

Nach dem Trocknen und Mahlen des Polymerisates wurde auf 150-850 μm Teilchengröße abgesiebt und 100 g des Pulvers

a) unter kräftigem Rühren mit einer Lösung aus 1 g Ethylenglykol, 2,5 g Wasser und 0,5 g Aluminiumsulfat-14-Hydrat vermischt und anschließend für 60 min. in einem Ofen, der auf 175 °C temperiert war, erhitzt.

| Produkt | TB | AAP$_{0.7}$ | SFC | TB+SFC |
|---|---|---|---|---|
| | [g/g] | [g/g] | [$10^{-7}$cm$^3$ s/g] | |
| Pulver (A) | 35,5 | | | |
| Beispiel 1 | 28,5 | 25 | 65 | 93,5 |

Vergleichsbeispiele 1 - 8

100 g des Polymerpulver A, bzw. Pulver B, Favor® SXM 6860 (siehe Vergleichsbeispiel 13) werden mit den im folgenden genannten Lösungen unter gründlichem Mischen beschichtet und anschließend getrocknet (60 °C, 60 min.) vgl. EP 0 233 067.

Lösung A: 25g einer Lösung aus Polyethylenglykol 400 (8 Teile), AlCl$_3$ x 6 H$_2$O (20 Teile) und Wasser (72 Teile).
Lösung B: 25g einer Lösung aus Polyethylenglykol 400 (8 Teile), Al$_2$(SO$_4$)$_3$ x 14 H$_2$O (20 Teile) und Wasser (72 Teile).
Lösung C: 25g einer Lösung aus 1,3-Butandiol (8 Teile), AlCl$_3$ x 6 H$_2$O (20 Teile) und Wasser (72 Teile)..
Lösung D: 25g einer Lösung aus 1,3-Butandiol (8 Teile), Al$_2$(SO$_4$)$_3$ x 14 H$_2$O (20 Teile) und Wasser (72 Teile).

| Vergleichsbeispiel (Pulver) | Lösung | TB [g/g] | AAP$_{0.7}$ [g/g] | SFC [$10^{-7}$ cm$^3$ s/g] | TB + SFC |
|---|---|---|---|---|---|
| 1 (A) | A | 26 | 15 | 1 | 27 |
| 2 (B) | A | 27 | 19 | 10 | 37 |
| 3 (A) | B | 26 | 14 | 7 | 33 |
| 4 (B) | B | 27 | 19 | 13 | 40 |
| 5 (A) | C | 26 | 15 | 7 | 33 |
| 6 (B) | C | 27 | 17 | 15 | 42 |
| 7 (A) | D | 26 | 15 | 8 | 34 |
| 8 (B) | D | 26 | 18 | 20 | 46 |

**Vergleichsbeispiele 9 - 12**

100 g des Polymerpulver A, bzw. des Pulvers B, Favor® SXM 6860 (siehe Vergleichsbeispiel 13) werden mit den im folgenden genannten Lösungen unter gründlichem Mischen beschichtet und anschließend getrocknet (100 °C, 90 min.) vgl. EP 0 233 067.

Lösung E: 50g einer Lösung aus 1,3-Butandiol (15 Teile), AlCl$_3$ x 6 H$_2$O (31 Teile) und Wasser (85 Teile).
Lösung F: 50g einer Lösung aus 1,3-Butandiol (15 Teile), Al$_2$(SO$_4$)$_3$ x 14 H$_2$O (31 Teile) und Wasser (85 Teile).

| Vergleichsbeispiel (Pulver) | Lösung | TB [g/g] | AAP$_{0.7}$ [g/g] | SFC [$10^{-7}$cm$^3$ s/g] | TB + SFC |
|---|---|---|---|---|---|
| 9 (A) | E | 25 | 16 | 2 | 27 |
| 10 (B) | E | 17 | 16 | 10 | 27 |
| 11 (A) | F | 24 | 17 | 18 | 42 |
| 12 (B) | F | 24 | 15 | 42 | 66 |

**Vergleichsbeispiel 13**

100g Favor® SXM 6860 (Handelsprodukt der Firma Stockhausen GmbH & Co., Oberflächen nachvernetztes Polyacrylat) wird unter kräftigem Rühren mit einer Lösung aus 2,5 g Wasser und 0,5 g Aluminiumsulfat-14-Hydrat vermischt und anschließend für 30 min. in einem Ofen, der auf 180 °C temperiert war, erhitzt.

|  | TB [g/g] | AAP$_{0.7}$ [g/g] | SFC [$10^{-7}$cm$^3$ s/g] | TB+SFC |
|---|---|---|---|---|
| **Pulver B** | 31,5 | 25,5 | 5 | 36,5 |
| **Vergleichsbeispiel 13** | 27 | 21,5 | 15 | 43 |

**Beispiel 2**

Eine pulverförmige, mit 0,8 Gew.% Polyethylenglykoldiacrylat, bezogen auf Acrylsäure, vernetzte Polyacrylsäure (Pulver C, 100 g), die bis 70 Mol-% als Natriumsalz neutralisiert vorlag, wurde nach dem Trocknen und Mahlen auf 150-850 µm abgesiebt und unter kräftigem Rühren mit einer Lösung aus 1 g Ethylenglycol, 2,5 g Wasser und 0,5 g Aluminiumsulfat-18-Hydrat vermischt und anschließend für 30 min. in einem Ofen, der auf 180 °C temperiert war, erhitzt.

| Produkt | TB [g/g] | AAP$_{0.7}$ [g/g] | SFC [$10^{-7}$cm$^3$ s/g] | TB+SFC |
|---|---|---|---|---|
| **Pulver C** | 32,5 | 10 | 0 | 32,5 |
| **Bespiel 2** | 28,5 | 23,0 | 50 | 78,5 |

**Beispiele 3 und 4:**

Jeweils 100 g einer pulverförmigen, vernetzten Polyacrylsäure (Pulver D), die zu 70 Mol.% als Natriumsalz vorlagen, wurden nach dem Trocknen und Mahlen auf 150 bis 180 µm abgesiebt und mit Lösungen, deren Zusammensetzung in der nachfolgenden Tabelle angegeben sind, unter kräftigem Rühren vermischt und anschließend in einem Ofen, entsprechend der unten angegebenen Bedingungen, erhitzt:

| Produkt | Al$_2$(SO$_4$)$_3$*** [g] | Glykol* [g] | H$_2$O [g] | TB [g/g] | AAP$_{0.7}$ [g/g] | SFC [$10^{-7}$ cm$^3$s/g] | TB+SFC | T/t [°C/min] |
|---|---|---|---|---|---|---|---|---|
| **Pulver D** |  |  |  | 31 | 10 | 0 | 31 |  |
| **Beispiel 3** | 0,5 | 1* | 3 | 26 | 22,5 | 95 | 121 | 180/30 |
| **Vergleichsbeispiel 14** |  | 1* | 3 | 26,5 | 24 | 42 | 68,5 | 180/30 |
| **Vergleichsbeispiel 15** |  | 1* | 3 | 30,5 | 10 | 0 | 30,5 | 149/60 |
| **Beispiel 4** | 0,5 | 0,8** | 3 | 26 | 23,5 | 83 | 109 | 185/40 |
| **Vergleichsbeispiel 16** |  | 0.8** | 3 | 26 | 24 | 53 | 79 | 185/40 |

*: Ethylenglykol
**: Propylenglykol
***: Al$_2$(SO$_4$)$_3$18H$_2$O

Die Beispiele zeigen die deutliche Verbesserung der Permeabilität der erfindungsgemäßen Polymerisate im gequollenen Zustand, gekennzeichnet durch einen hohen SFC-Wert. Die beiden anderen relevanten Parameter, die Teebeutelretention und die Flüssigkeitsaufnahme unter Druck (AAP$_{0.7}$) liegen trotz hoher Permeabilität auf hohem Niveau. Gezeigt wurde auch, daß sich durch die Behandlung mit einer Kombination aus Polyol und einer anorganischen Salzkomponente mit Erhitzen des beschichteten Polymerisates auf mindestens 150°C eine entsprechende Eigenschaftskombination aus hohem Retentionsvermögen, guter Flüssigkeitsaufnahmefähigkeit unter Druck und hoher Permeabilität im gequollenen Zustand erreichen läßt. Eine Verwendung von nur der Salzkomponente . (Vergleichsbeispiele 13) oder der Vernetzung bei einer Reaktionstemperatur unter den erfindungsgemäß, (Vergleichsbeispiele 1 - 12 und 14) führt nicht zum gewünschten Eigenschaftsprofil. Die gemäß den Vergleichsbeispielen erhaltenen Produkte führen auch nicht annähernd zu Superabsorbern, die mit den erfindungsgemäß verwendeten Produkten vergleichbar sind. Darüber hinaus treten bei der Beschichtung der Polymerisate mit großen Mengen wässriger Lösung, bzw. organischer Lösungsmittel erhebliche Probleme bezüglich der Durchführbarkeit der Verfahren auf (starkes Verklumpen des Materials, bzw. große Mengen abzuführender organischer Dämpfe).

**Patentansprüche**

1. Verwendung eines pulverförmigen, an der Oberfläche nachvernetzten, Wasser, wäßrige oder seröse Flüssigkeiten sowie Blut absorbierenden Polymerisates, aufgebaut aus

   a) 55-99,9 Gew% polymerisierten, ethylenisch ungesättigten, säuregruppenenthaltenden Monomeren, die zu mindestens 25 Mol% neutralisiert sind,
   b) 0-40 Gew.-% polymerisierten, ethylenisch ungesättigten, mit a) copolymerisierbaren Monomeren,
   c) 0,1 - 5,0 Gew.-% eines oder mehrerer polymerisierter Vernetzer,
   d) 0-30 Gew.-% eines wasserlöslichen Polymeren
   wobei die Summe der Gewichtsmengen a) bis d) 100 Gew.% beträgt, als Absorptionsmittel in Windeln, Damenbinden oder Wundabdeckungen, **dadurch gekennzeichnet, daß** das Polymerisat erhältlich ist durch das Beschichten des Polymerisates mit
   e) 0,01 bis 5 Gew.-%, bezogen auf das Polymerisat, wenigstens eines Polyols als Oberflächennachvernetzungsmittel in Form einer wässrigen Lösung und mit
   f) 0,001 bis 0,5 Gew.-%, bezogen auf das Polymerisat, eines Kations in Form einer wässrigen Lösung

   und das Erhitzen auf eine Nachvernetzungstemperatur von 150 bis 300°C
   und

   - bei einer Permeabilität (SFC) bis zu $70 \cdot 10^{-7}$ s $cm^3$/g eine Retention (TB) von mindestens 27 g/g aufweist,
   - bei einer Permeabilität (SFC) von > 70 bis $150 \cdot 10^{-7}$ s $cm^3$/g eine Retention (TB) von mindestens 25 g/g aufweist.

2. Verwendung nach Anspruch 1, **dadurch gekennzeichnet, dass** das Polymerisat erhalten ist durch das Beschichten des Polymerisates mit

   e) 0,01 bis 5 Gew.-%, bezogen auf das Polymerisat, wenigstens eines Polyols als Oberflächennachvernetzungsmittel in Form einer wässrigen Lösung und mit
   f) 0,001 bis 0,5 Gew.-%, bezogen auf das Polymerisat, eines Kations in Form einer wässrigen Lösung

   und das Erhitzen auf eine Nachvernetzungstemperatur von 150 bis 300°C.

3. Eine Windel beinhaltend eine pulverförmiges, an der Oberfläche nachvemetztes, Wasser, wäßrige oder seröse Flüssigkeiten sowie Blut absorbierendes Polymerisat, aufgebaut aus

   a) 55-99,9 Gew% polymerisierten, ethylenisch ungesättigten, säuregruppenenthaltenden Monomeren, die zu mindestens 25 Mol% neutralisiert sind,
   b) 0-40 Gew.-% polymerisierten, ethylenisch ungesättigten, mit a) copolymerisierbaren Monomeren,
   c) 0,1 - 5,0 Gew.-% eines oder mehrerer polymerisierter Vernetzer,
   d) 0-30 Gew.-% eines wasserlöslichen Polymeren
   wobei die Summe der Gewichtsmengen a) bis d) 100 Gew.% beträgt, **dadurch gekennzeichnet, daß** das Polymerisat erhältlich ist durch das Beschichten des Polymerisates mit
   e) 0,01 bis 5 Gew.-%, bezogen auf das Polymerisat, wenigstens eines Polyols als Oberflächennachvernetzungsmittel in Form einer wässrigen Lösung und mit
   f) 0,001 bis 0,5 Gew.-%, bezogen auf das Polymerisat, eines Kations in Form einer wässrigen Lösung

   und das Erhitzen auf eine Nachvernetzungstemperatur von 150 bis 300°C und

   - bei einer Permeabilität (SFC) bis zu $70 \cdot 10^{-7}$ s $cm^3$/g eine Retention (TB) von mindestens 27 g/g aufweist,
   - bei einer Permeabilität (SFC) von > 70 bis $150 \cdot 10^{-7}$ s $cm^3$/g eine Retention (TB) von mindestens 25 g/g aufweist.

4. Eine Damenbinde beinhaltend eine pulverformiges, an der Oberfläche nachvemetztes, Wasser, wäßrige oder seröse Flüssigkeiten sowie Blut absorbierendes Polymerisat, aufgebaut aus

   a) 55-99,9 Gew% polymerisierten, ethylenisch ungesättigten, säuregruppenenthaltenden Monomeren, die zu mindestens 25 Mol% neutralisiert sind,
   b) 0-40 Gew.-% polymerisierten, ethylenisch ungesättigten, mit a) copolymerisierbaren Monomeren,
   c) 0,1 - 5,0 Gew.-% eines oder mehrerer polymerisierter Vernetzer,

d) 0-30 Gew.-% eines wasserlöslichen Polymeren
wobei die Summe der Gewichtsmengen a) bis d) 100 Gew.% beträgt, **dadurch gekennzeichnet, daß** das Polymerisat erhältlich ist durch das Beschichten des Polymerisates mit
e) 0,01 bis 5 Gew.-%, bezogen auf das Polymerisat, wenigstens eines Polyols als Oberflächennachvernetzungsmittel in Form einer wässrigen Lösung und mit
f) 0,001 bis 0,5 Gew.-%, bezogen auf das Polymerisat, eines Kations in Form einer wässrigen Lösung

und das Erhitzen auf eine Nachvernetzungstemperatur von 150 bis 300°C und

- bei einer Permeabilität (SFC) bis zu 70 · $10^{-7}$ s $cm^3$/g eine Retention (TB) von mindestens 27 g/g aufweist,
- bei einer Permeabilität (SFC) von > 70 bis 150 · $10^{-7}$ s $cm^3$/g eine Retention (TB) von mindestens 25 g/g aufweist.

5. Eine Wundabdeckung beinhaltend eine pulverförmiges, an der Oberfläche nachvernetztes, Wasser, wäßrige oder seröse Flüssigkeiten sowie Blut absorbierendes Polymerisat, aufgebaut aus

a) 55-99,9 Gew% polymerisierten, ethylenisch ungesättigten, säuregruppenenthaltenden Monomeren, die zu mindestens 25 Mol% neutralisiert sind,
b) 0-40 Gew.-% polymerisierten, ethylenisch ungesättigten, mit a) copolymerisierbaren Monomeren,
c) 0,1 - 5,0 Gew.-% eines oder mehrerer polymerisierter Vernetzer,
d) 0-30 Gew.-% eines wasserlöslichen Polymeren
wobei die Summe der Gewichtsmengen a) bis d) 100 Gew.% beträgt, **dadurch gekennzeichnet, daß** das Polymerisat erhältlich ist durch das Beschichten des Polymerisates mit
e) 0,01 bis 5 Gew.-%, bezogen auf das Polymerisat, wenigstens eines Polyols als Oberflächennachvernetzungsmittel in Form einer wässrigen Lösung und mit
f) 0,001 bis 0,5 Gew.-%, bezogen auf das Polymerisat, eines Kations in Form einer wässrigen Lösung

und das Erhitzen auf eine Nachvernetzungstemperatur von 150 bis 300°C und

- bei einer Permeabilität (SFC) bis zu 70 · $10^{-7}$ s $cm^3$/g eine Retention (TB) von mindestens 27 g/g aufweist,
- bei einer Permeabilität (SFC) von > 70 bis 150 · $10^{-7}$ s $cm^3$/g eine Retention (TB) von mindestens 25 g/g aufweist.

**Claims**

1. Use of a pulverulent, surface postcrosslinked polymer that absorbs water, aqueous or serous fluids and blood, formed from

a) 55-99.9% by weight of polymerized, ethylenically unsaturated monomers that contain acid groups and have been neutralized to an extent of at least 25 mol%,
b) 0-40% by weight of polymerized, ethylenically unsaturated monomers copolymerizable with a),
c) 0.1-5.0% by weight of one or more polymerized crosslinkers,
d) 0-30% by weight of a water-soluble polymer, where the sum total of the amounts by weight a) to d) is 100% by weight, as absorbent in nappies, sanitary towels or wound dressings, **characterized in that** the polymer is obtainable by coating the polymer with
e) 0.01% to 5% by weight, based on the polymer, for at least one polyol as surface postcrosslinker in the form of an aqueous solution and with
f) 0.001% to 0.5% by weight, based on the polymer, of a cation in the form of an aqueous solution, and heating to a postcrosslinking temperature of 150 to 300°C

and

- at a permeability (SFC) of up to 70 · $10^{-7}$ s $cm^3$/g has a retention (TB) of at least 27 g/g,
- at a permeability (SFC) of > 70 to 150 · $10^{-7}$ s $cm^3$/g has a retention (TB) of at least 25 g/g.

2. Use according to Claim 1, **characterized in that** the polymer has been obtained by the coating of the polymer with

e) 0.01% to 5% by weight, based on the polymer, of at least one polyol as surface postcrosslinker in the form of an aqueous solution and with

f) 0.001% to 0.5% by weight, based on the polymer, of a cation in the form of an aqueous solution,

and heating to a postcrosslinking temperature of 150 to 300°C.

**3.** A nappy comprising a pulverulent, surface postcrosslinked polymer that absorbs water, aqueous or serous fluids and blood, formed from

a) 55-99.9% by weight of polymerized, ethylenically unsaturated monomers that contain acid groups and have been neutralized to an extent of at least 25 mol%,
b) 0-40% by weight of polymerized, ethylenically unsaturated monomers copolymerizable with a),
c) 0.1-5.0% by weight of one or more polymerized crosslinkers,
d) 0-30% by weight of a water-soluble polymer,
where the sum total of the amounts by weight a) to d) is 100% by weight, **characterized in that** the polymer is obtainable by coating the polymer with
e) 0.01% to 5% by weight, based on the polymer, for at least one polyol as surface postcrosslinker in the form of an aqueous solution and with
f) 0.001% to 0.5% by weight, based on the polymer, of a cation in the form of an aqueous solution,

and heating to a postcrosslinking temperature of 150 to 300°C
and

- at a permeability (SFC) of up to 70 · $10^{-7}$ s $cm^3$/g has a retention (TB) of at least 27 g/g,
- at a permeability (SFC) of > 70 to 150 · $10^{-7}$ s $cm^3$/g has a retention (TB) of at least 25 g/g.

**4.** A sanitary towel comprising a pulverulent, surface postcrosslinked polymer that absorbs water, aqueous or serous fluids and blood, formed from

a) 55-99.9% by weight of polymerized, ethylenically unsaturated monomers that contain acid groups and have been neutralized to an extent of at least 25 mol%,
b) 0-40% by weight of polymerized, ethylenically unsaturated monomers copolymerizable with a),
c) 0.1-5.0% by weight of one or more polymerized crosslinkers,
d) 0-30% by weight of a water-soluble polymer,
where the sum total of the amounts by weight a) to d) is 100% by weight, **characterized in that** the polymer is obtainable by coating the polymer with
e) 0.01% to 5% by weight, based on the polymer, for at least one polyol as surface postcrosslinker in the form of an aqueous solution and with
f) 0.001% to 0.5% by weight, based on the polymer, of a cation in the form of an aqueous solution,

and heating to a postcrosslinking temperature of 150 to 300°C
and

- at a permeability (SFC) of up to 70 · $10^{-7}$ s $cm^3$/g has a retention (TB) of at least 27 g/g,
- at a permeability (SFC) of > 70 to 150 · $10^{-7}$ s $cm^3$/g has a retention (TB) of at least 25 g/g.

**5.** A wound dressing comprising a pulverulent, surface postcrosslinked polymer that absorbs water, aqueous or serous fluids and blood, formed from

a) 55-99.9% by weight of polymerized, ethylenically unsaturated monomers that contain acid groups and have been neutralized to an extent of at least 25 mol%,
b) 0-40% by weight of polymerized, ethylenically unsaturated monomers copolymerizable with a),
c) 0.1-5.0% by weight of one or more polymerized crosslinkers,
d) 0-30% by weight of a water-soluble polymer,
where the sum total of the amounts by weight a) to d) is 100% by weight, **characterized in that** the polymer is obtainable by coating the polymer with
e) 0.01% to 5% by weight, based on the polymer, for at least one polyol as surface postcrosslinker in the form of an aqueous solution and with
f) 0.001% to 0.5% by weight, based on the polymer, of a cation in the form of an aqueous solution,

and heating to a postcrosslinking temperature of 150 to 300°C
and

- at a permeability (SFC) of up to $70 \cdot 10^{-7}$ s cm$^3$/g has a retention (TB) of at least 27 g/g,
- at a permeability (SFC) of > 70 to $150 \cdot 10^{-7}$ s cm$^3$/g has a retention (TB) of at least 25 g/g.

## Revendications

1. Utilisation d'un polymère en poudre, post-réticulé en surface, absorbant l'eau, les liquides aqueux ou séreux, ainsi que le sang, formé par :

   a) 55 à 99,9 % en poids de monomères éthyléniquement insaturés contenant des groupes acides polymérisés, qui sont neutralisés à hauteur d'au moins 25 % en moles,
   b) 0 à 40 % en poids de monomères éthyléniquement insaturés copolymérisables avec a) polymérisés,
   c) 0,1 à 5,0 % en poids d'un ou de plusieurs agents de réticulation polymérisés,
   d) 0 à 30 % en poids d'un polymère soluble dans l'eau, la somme des quantités en poids de a) à d) étant de 100 % en poids, en tant qu'agent d'absorption dans des couches, des serviettes hygiéniques ou des pansements, **caractérisée en ce que** le polymère peut être obtenu par revêtement du polymère avec
   e) 0,01 à 5 % en poids, par rapport au polymère, d'au moins un polyol en tant qu'agent de post-réticulation de surface sous la forme d'une solution aqueuse, et avec
   f) 0,001 à 0,5 % en poids, par rapport au polymère, d'un cation sous la forme d'une solution aqueuse,

   et chauffage à une température de post-réticulation de 150 à 300 °C,
   et

   - présente à une perméabilité (SFC) de jusqu'à $70 \cdot 10^{-7}$ s cm$^3$/g une rétention (TB) d'au moins 27 g/g,
   - présente à une perméabilité (SFC) de > 70 à $150 \cdot 10^{-7}$ s cm$^3$/g une rétention (TB) d'au moins 25 g/g.

2. Utilisation selon la revendication 1, **caractérisée en ce que** le polymère est obtenu par revêtement du polymère avec

   e) 0,01 à 5 % en poids, par rapport au polymère, d'au moins un polyol en tant qu'agent de post-réticulation de surface sous la forme d'une solution aqueuse, et avec
   f) 0,001 à 0,5 % en poids, par rapport au polymère, d'un cation sous la forme d'une solution aqueuse,

   et chauffage à une température de post-réticulation de 150 à 300 °C.

3. Couche contenant un polymère en poudre, post-réticulé en surface, absorbant l'eau, les liquides aqueux ou séreux, ainsi que le sang, formé par :

   a) 55 à 99,9 % en poids de monomères éthyléniquement insaturés contenant des groupes acides polymérisés, qui sont neutralisés à hauteur d'au moins 25 % en moles,
   b) 0 à 40 % en poids de monomères éthyléniquement insaturés copolymérisables avec a) polymérisés,
   c) 0,1 à 5,0 % en poids d'un ou de plusieurs agents de réticulation polymérisés,
   d) 0 à 30 % en poids d'un polymère soluble dans l'eau, la somme des quantités en poids de a) à d) étant de 100 % en poids, **caractérisée en ce que** le polymère peut être obtenu par revêtement du polymère avec
   e) 0,01 à 5 % en poids, par rapport au polymère, d'au moins un polyol en tant qu'agent de post-réticulation de surface sous la forme d'une solution aqueuse, et avec
   f) 0,001 à 0,5 % en poids, par rapport au polymère, d'un cation sous la forme d'une solution aqueuse,

   et chauffage à une température de post-réticulation de 150 à 300 °C,
   et

   - présente à une perméabilité (SFC) de jusqu'à $70 \cdot 10^{-7}$ s cm$^3$/g une rétention (TB) d'au moins 27 g/g,
   - présente à une perméabilité (SFC) de > 70 à $150 \cdot 10^{-7}$ s cm$^3$/g une rétention (TB) d'au moins 25 g/g.

4. Serviette hygiénique contenant un polymère en poudre, post-réticulé en surface, absorbant l'eau, les liquides aqueux ou séreux, ainsi que le sang, formé par :

a) 55 à 99,9 % en poids de monomères éthyléniquement insaturés contenant des groupes acides polymérisés, qui sont neutralisés à hauteur d'au moins 25 % en moles,
b) 0 à 40 % en poids de monomères éthyléniquement insaturés copolymérisables avec a) polymérisés,
c) 0,1 à 5,0 % en poids d'un ou de plusieurs agents de réticulation polymérisés,
d) 0 à 30 % en poids d'un polymère soluble dans l'eau, la somme des quantités en poids de a) à d) étant de 100 % en poids, **caractérisée en ce que** le polymère peut être obtenu par revêtement du polymère avec
e) 0,01 à 5 % en poids, par rapport au polymère, d'au moins un polyol en tant qu'agent de post-réticulation de surface sous la forme d'une solution aqueuse, et avec
f) 0,001 à 0,5 % en poids, par rapport au polymère, d'un cation sous la forme d'une solution aqueuse,

et chauffage à une température de post-réticulation de 150 à 300 °C,
et

- présente à une perméabilité (SFC) de jusqu'à $70 \cdot 10^{-7}$ s $cm^3$/g une rétention (TB) d'au moins 27 g/g,
- présente à une perméabilité (SFC) de > 70 à $150 \cdot 10^{-7}$ s $cm^3$/g une rétention (TB) d'au moins 25 g/g.

5. Pansement contenant un polymère en poudre, post-réticulé en surface, absorbant l'eau, les liquides aqueux ou séreux, ainsi que le sang, formé par :

a) 55 à 99,9 % en poids de monomères éthyléniquement insaturés contenant des groupes acides polymérisés, qui sont neutralisés à hauteur d'au moins 25 % en moles,
b) 0 à 40 % en poids de monomères éthyléniquement insaturés copolymérisables avec a) polymérisés,
c) 0,1 à 5,0 % en poids d'un ou de plusieurs agents de réticulation polymérisés,
d) 0 à 30 % en poids d'un polymère soluble dans l'eau, la somme des quantités en poids de a) à d) étant de 100 % en poids, **caractérisé en ce que** le polymère peut être obtenu par revêtement du polymère avec
e) 0,01 à 5 % en poids, par rapport au polymère, d'au moins un polyol en tant qu'agent de post-réticulation de surface sous la forme d'une solution aqueuse, et avec
f) 0,001 à 0,5 % en poids, par rapport au polymère, d'un cation sous la forme d'une solution aqueuse,

et chauffage à une température de post-réticulation de 150 à 300 °C,
et

- présente à une perméabilité (SFC) de jusqu'à $70 \cdot 10^{-7}$ s $cm^3$/g une rétention (TB) d'au moins 27 g/g,
- présente à une perméabilité (SFC) de > 70 à $150 \cdot 10^{-7}$ s $cm^3$/g une rétention (TB) d'au moins 25 g/g.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19646484
- US 4043952 A
- DE 4020780 A
- EP 0574260 A
- EP 0233067 A
- WO 9605234 A
- WO 9522356 A
- WO 9712575 A

- US 4286082 A
- DE 2706135
- US 4076663 A
- US 4340706 A
- DE 3713601
- DE 2840010
- EP 0339461 A